# EUROPEAN PATENT SPECIFICATION

(11) **EP 1 333 805 B1**
(45) Date of publication and mention of the grant of the patent: **05.04.2006**
(21) Application number: 01996350.3
(22) Date of filing: 02.11.2001
(51) Int. Cl.: A61Q 19/08, A61Q 19/00, A61K 8/49

(54) **COSMETIC METHOD OF TREATING SKIN**
KOSMETISCHE HAUTBEHANDLUNGSMETHODE
PROCEDE COSMETIQUE POUR LE TRAITEMENT DE LA PEAU

(30) Priority: 14.11.2000 GB 0027769
(43) Date of publication of application: 13.08.2003
(73) Proprietor: UNILEVER PLC, London EC4P 4BQ (GB); UNILEVER N.V., 3013 AL Rotterdam (NL)
(72) Inventor: DONOVAN, Robert M., c/o Unilever Research Colworth, Bedford, Bedfordshire MK44 1LQ (GB); GREEN, Martin R., c/o Unilever Research Colworth, Bedford, Bedfordshire MK44 1LQ (GB); TASKER, Maria, C., c/o Unilever Research Colworth, Bedford, Bedfordshire MK44 1LQ (GB); YATES, Paula, R., c/o Unilever Research Colworth, Bedford, Bedfordshire MK44 1LQ (GB)
(74) Representative: Acham, Nicholas Clive
(86) International application number: PCT/EP2001/012732
(87) International publication number: WO 2002/039960

(56) References cited:
- EP-A- 1 190 630
- DE-A- 19 922 287
- US-A- 6 071 525
- PATENT ABSTRACTS OF JAPAN vol. 1996, no. 10, 31 October 1996 (1996-10-31) & JP 08 165238 A (SANKYO CO LTD), 25 June 1996 (1996-06-25)
- DATABASE WPI Section Ch, Week 197713 Derwent Publications Ltd., London, GB; Class D21, AN 1977-22800Y XP002198821 & JP 52 021339 A (MATSUI T), 17 February 1977 (1977-02-17)
- MILLIGAN S R ET AL: "The endocrine activities of 8-prenylnaringenin and related hop (Humulus lupulus L.) flavonoids" BIOSIS, XP002185291
- PATENT ABSTRACTS OF JAPAN vol. 1999, no. 14, 22 December 1999 (1999-12-22) & JP 11 246387 A (SHISEIDO CO LTD), 14 September 1999 (1999-09-14)
- PATENT ABSTRACTS OF JAPAN vol. 015, no. 227 (C-0839), 10 June 1991 (1991-06-10) & JP 03 068518 A (TSUMURA & CO), 25 March 1991 (1991-03-25)
- PATENT ABSTRACTS OF JAPAN vol. 015, no. 112 (C-0815), 18 March 1991 (1991-03-18) & JP 03 005423 A (ICHIMARU PHARCOS CO LTD), 11 January 1991 (1991-01-11)

## Description

This invention relates to a cosmetic method of improving the condition and appearance of skin and to the use of 8-prenylnaringenin in the preparation of topical compositions for improving the condition and appearance of skin.

Skin is subject to deterioration through dermatological disorders, environmental abuse (wind, air conditioning, central heating, etc.) or through the normal aging process (chronoaging) which may be accelerated by exposure of skin to sun (photoaging). In recent years the demand for cosmetic compositions and cosmetic methods for improving the appearance and condition of skin has grown enormously.

Consumers are increasingly seeking "anti-aging" cosmetic products which treat or delay the visible signs of chronoaging and photoaging skin such as wrinkles, lines, sagging, hyperpigmentation and age spots.

Consumers also frequently seek other benefits from cosmetic products in addition to anti-aging. The concept of "sensitive skin" has also raised the consumer demand for cosmetic products which improve the appearance and condition of sensitive, dry and/or flaky skin and to soothe red, and/or irritated skin. Consumers also desire cosmetic products which treat spots, pimples, blemishes etc.

8-prenylnaringenin as a substance is known. For example, in The Journal of Endocrinology & Metabolism Vol. 83 No. 6, Milligan et al., " Identification of a potent phytoestrogen in hops and beer", 8-prenylnaringenin is recognized as being a potent phytoestrogen. The structure of 8-prenylnaringenin is also shown in that paper. 8-prenylnaringenin occurs naturally in hops, from which it can be isolated and concentrated.

We have now surprisingly found further undisclosed properties of 8-prenylnaringenin, which are useful in cosmetic compositions for topical application to skin to provide previously undisclosed skin care benefits.

We have now found that effective treatment and prevention of normal skin conditions due to chronoaging or photoaging, such as wrinkles, lines, sagging, hyperpigmentation and age spots, may be obtained through the application of cosmetic compositions to the skin which comprise 8-prenylnaringenin or derivatives thereof. We have also found that the use of 8-prenylnaringenin in cosmetic compositions advantageously provides further skin benefits in addition to anti-aging such as for soothing sensitive and/or irritated skin.

In a first aspect the invention comprises a topical composition for application to the human skin comprising an effective amount of 8-prenylnaringenin.

According to a further aspect of the present invention there is a method of providing at least one cosmetic skin care benefit selected from: treating/preventing wrinkling, sagging, aged and/or photodamaged skin; boosting collagen deposition in skin, boosting decorin production in skin; soothing irritated, red and/or sensitive skin; improving skin texture, smoothness and/or firmness; the method comprising applying to the skin a topical composition comprising 8-prenylnaringenin and/or derivatives thereof.

The present invention also encompasses the use of 8-prenylnaringenin and/or derivatives thereof in the preparation of a topical composition for providing at least one skin care benefit selected from treating/preventing wrinkling, sagging, aged and/or photodamaged skin; boosting collagen deposition in skin, boosting decorin production in skin; soothing irritated, red and/or sensitive skin; improving skin texture, smoothness and/or firmness.

Another aspect of the present invention provides 8-prenylnaringenin for use in the treatment of wrinkling, sagging, aged and/or photodamaged skin; for boosting collagen deposition in skin; for boosting decorin production in skin; for soothing irritated, red and/or sensitive skin; and for improving skin texture, smoothness and/or firmness.

The inventive methods and use of 8-prenylnaringenin thus provide anti-aging benefits which result in the promotion of smooth and supple skin with improved elasticity and a reduced or delayed appearance of wrinkles and aged skin, with improved skin colour. A general improvement in the appearance, texture and condition, in particular with respect to the radiance, clarity, and general youthful appearance of skin may be achieved. The inventive methods and uses are also beneficial for soothing and calming sensitive and/or irritated skin. Thus the inventive methods advantageously provide a wide range of skin care benefits.

These benefits might include improved hydration, texture/tone, smoothness, silkiness, firmness, strength/resilience and radiance.

The term "treating" as used herein includes within its scope reducing, delaying and/or preventing the above mentioned skin conditions such as wrinkled, aged, photodamaged, and/or irritated skin and generally enhancing the quality of skin and improving its appearance and texture by preventing or reducing wrinkling and increasing flexibility, firmness, smoothness, suppleness and elasticity of the skin. The cosmetic methods and the uses of 8-prenylnaringenin and/or derivatives according to the invention may be useful for treating skin which is already in a wrinkled, aged, photo-damaged and irritated condition or for treating youthful skin to prevent or reduce those aforementioned deteriorative changes due to the normal aging/photo aging process.

The invention also includes derivatives of the free molecule which thus comprise 8-prenylnaringenin moieties.

The active 8-prenylnaringenin to be employed in accordance with the present invention is present in the topical composition in an effective amount. Normally the total amount of the active is present in an amount between 0.0001% and 50% by weight of the composition. More preferably the amount is from 0.001% to 10% and most preferably from 0.01% to 1% in order to maximize benefits at a minimum cost.

The composition used according to the invention also comprises a dermatologically/cosmetically acceptable vehicle to act as a dilutant, dispersant or carrier for the active. The vehicle may comprise materials commonly employed in skin care products such as water, liquid or solid emollients, silicone oils, emulsifiers, solvents, humectants, thickeners, powders, propellants and the like.

The vehicle will usually form from 5% to 99.9%, preferably from 25% to 80% by weight of the composition, and can, in the absence of other cosmetic adjuncts, form the balance of the composition.

Besides the active, 8-prenylnaringenin, other specific skin-benefit actives such as sunscreens, skin lightening agents and skin tanning agents may also be included. The vehicle may also further include adjuncts such as perfumes, opacifiers, preservatives, colourants and buffers.

To prepare the topical composition used in the method of the present invention, the usual manner for preparing skin care products may be employed. The active components are generally incorporated in a dermatologically acceptable carrier in conventional manner. The active components can suitably first be dissolved or dispersed in a portion of the water or another solvent or liquid to be incorporated in the composition. The preferred compositions are oil-in-water or water-in-oil emulsions.

The composition may be in the form of conventional skin-care products such as a cream, gel or lotion or the like. The composition can also be in the form of a so-called "wash-off" product e.g. a bath or shower gel, possibly containing a delivery system for the actives to promote adherence to the skin during rinsing. Most preferably the product is a "leave-on" product; a product to be applied to the skin without a deliberate rinsing step soon after its application to the skin.

The composition may packaged in any suitable manner such as in a jar, a bottle, tube, roll-ball, or the like, in the conventional manner.

The method of the present invention may be carried out one or more times daily to the skin which requires treatment. The improvement in skin appearance will usually become visible after 1 to 3 months, depending on skin condition, the concentration of the active components used in the inventive method, the amount of composition used and the frequency with which it is applied. In general, a small quantity of the composition, for example from 0.1 to 5 ml is applied to the skin from a suitable container or applicator and spread over and/or rubbed into the skin using the hands or fingers or a suitable device. A rinsing step may optionally follow depending on whether the composition is formulated as a "leave-on" or a "rinse-off" product.

In order that the present invention may be more readily understood, the following examples are given, by way of illustration only, with reference to the accompanying drawings, in which:
- Figure 1 shows the effect of 8-prenylnaringenin on decorin synthesis;
- Figure 2 shows the effect of 8-prenylnaringenin on procollagen synthesis; and
- Figure 3 shows the effect of 8-prenylnaringenin on the reduction of PGE2 expression.

### EXAMPLES

The first example demonstrates the anti-aging benefits of 8-prenylnaringenin.

It is known from our co-pending European application no. 99908956.8 that topical retinoic acid treatments can be used to cause upregulation of procollagen I and decorin in vivo.

### Example 1

### Procedure For Measuring Procollagen-I and Decorin Synthesis In Human Dermal Fibroblasts

### Preparation of Dermal Fibroblast Conditioned Medium

Primary human foreskin fibroblasts at passage 2 (P2) were seeded into 12-well plates at 10000 cells/cm² and maintained for 24 hours in an atmosphere of 5% carbon dioxide and 4% oxygen in Dulbeccos Modified Eagles Medium (DMEM) supplemented with 10% foetal calf serum. After this time the cells were washed with serum free DMEM and then incubated in fresh serum free DMEM for a further 60 hours. The fibroblast monolayers were then washed again with serum free DMEM. Test reagents and vehicle controls were added to the cells in triplicate in a final volume of 0.4ml/well fresh serum free DMEM and incubated for a further 24 hours. This fibroblast conditioned medium was either analyzed immediately or snap frozen in liquid nitrogen and stored at -70°C for future analysis. The cells were then counted and data from the dot-blot analysis subsequently standardized to cell number.

### Dot Blot Assay for Procollagen-I and Decorin Protein in Dermal Fibroblast Conditioned Medium

Samples of conditioned medium from dermal fibroblasts treated with vehicle (as a control) or test reagents were supplemented with 20mM dithiothreitol (1:10 dilution of 200mM stock solution) and 0.1% sodium dodecylsulphate (1:100 dilution of 10% stock solution), mixed well and then incubated at 75°C for 2 minutes. A standard for the assay was generated by serial dilution of neat fibroblast conditioned medium from fibroblasts seeded at 10000 cells/cm² in a 175cm² flask and maintained in serum free DMEM as described above.

Assay samples were subsequently applied in triplicate to a pre-wetted sheet of Immobilon-P transfer membrane using the 96-well Bio-Dot Apparatus from Bio-Rad as described in the manufacturer's guidelines. Approximately 200µl of medium was applied per well. The medium was allowed to filter through the membrane under gravity (30 minutes) after which the membrane was washed twice with PBS (200µl). These PBS washes were allowed to filter through the membrane under gravity (2x15 minutes). The Bio-Dot apparatus was then attached to a vacuum manifold and a third and final PBS wash carried out under suction. The apparatus was disassembled, the membrane removed and quickly cut as required before being placed in blocking buffer overnight at 4°C.

Membranes prepared for decorin analysis were blocked with 3% (w/v) BSA/ 0.1% (v/v) Tween 20 in PBS, whilst those for procollagen-I analysis were blocked with 5% (w/v) non fat dried milk powder/ 0.05% Tween 20 in PBS. The following day, the membranes were probed with 1:10000 dilution of primary antibodies to either human procollagen-1 (MAB1912; rat monoclonal; Chemicon Int. Inc., Temecula, CA) or human decorin (rabbit polyclonal; Biogenesis) for 2 hours at room temperature. The membranes were subsequently washed with TBS/ 0.05% Tween 20 (3 x 5 minutes) and then incubated with 1:1000 dilution of ¹²⁵I-conjugated anti-rat or anti-rabbit F(ab')2 fragments (Amersham) as required for 1 hour at room temperature.

Following this the Immobilon strips were again washed with TBS/Tween 20 (3 x 5 minutes) before being allowed to dry in air at room temperature. The dried membranes were wrapped in cellophane and exposed to a Molecular Dynamics storage phosphor screen for 16-18 hours. At the end of this time the exposed screen was scanned by a phosphorimager (Molecular Dynamics Phosphorimager SF) using ImageQuant™ software. Dot intensity was assessed by computer-assisted image analysis using the quantification tools in ImageQuant™, standardized to cell number and the effects of various test reagents on decorin and procollagen-I synthesis were determined relative to a vehicle treated control value of 100 arbitrary units.

### TESTS

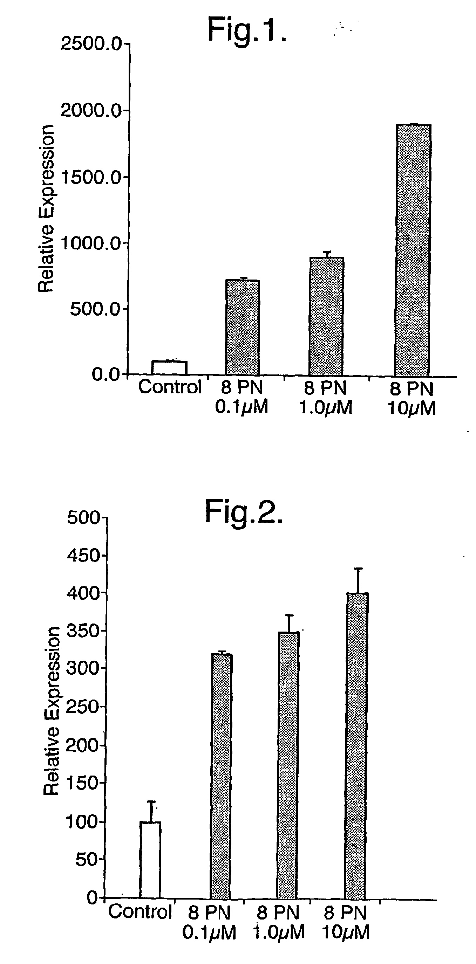

The results of the assays are shown graphically in figures 1 and 2. In order to normalize the results the effects of the test substance was determined relative to a vehicle treated control value of 100 arbitrary units.

For comparison, a trial was performed with retinoic acid to assess its effect on decorin synthesis in human dermal fibroblasts.

The results in figures 1 and 2 indicate that 8-prenylnaringenin significantly upregulates the synthesis of both procollagen-I and decorin in human dermal fibroblasts as compared to the control.

The level of decorin in skin is associated with improved condition and appearance of skin. Increasing the level of decorin in skin is important for controlled and correct deposition of collagen in skin which is associated with many skin benefits such as wrinkle effacement and dermal repair of photodamaged skin.

The comparative trial with retinoic acid (1µm) showed an upregluation of decorin, 138 3 14(p=0.035, n=4), as determined relative to a vehicle treated control value of 100 arbitrary units. Surprisingly, the data thus further indicates that the magnitude of the upregulation of and decorin synthesis in human dermal fibroblasts effected by exceeds that of the bench-mark anti-aging dermal repair active, retinoic acid.

Further comparative tests were conducted to compare the upregulation of Procollagen I and Decorin using 8-prenylnaringenin and other known estrogens, or phytoestrogens.

The same protocols as described above were used in the tests.

Initially, results were obtained for the known estrogens/phytoestrogens in media which contained Phenol Red (itself having estrogenic activity). The tests were then repeated for 8-prenylnaringenin and the known estrogens/phytoestrogens in media which was free of Phenol Red. The results are set out below in Tables 1 and 2.

**Table 1 Percentage increases in the levels of Procollagen I and Decorin secreted from human fibroblasts cultured in media containing phenol red.**

| Active at 1 uM (in media containing phenol red) | Procollagen I | Decorin |
|---|---|---|
| 17-β-Estradiol | 107.7 | 121 |
| Resveratrol | 102.1 | 113.1 |
| Daidzein | 118 | 98.9 |
| Genistein | 124 | 114 |

Data are presented in arbitrary units relative to a vehicle control value of 100.

**Table 2 Percentage increases in the levels of Procollagen I and Decorin secreted from human fibroblasts cultured in media minus phenol red.**

| Active at 1 uM (phenol red-free media) | Procollagen I | Decorin |
|---|---|---|
| 8-Prenylnarningenin | 350 | 800 |
| 17-β-Estradiol | 170 | 130 |
| Daidzein | 152 | 252 |
| Genistein | 200 | 240 |

Data are presented in arbitrary units relative to a vehicle control value of 100.

From the above test results it is clear that 8-Prenylnaringenin is a much better inducer of Procollagen I and Decorin than other known estrogens or phytoestrogens themselves. Without wishing to be bound by theory, it is believed that 8-Prenylnaringenin is not acting like a phytoestrogen, but may be promoting the synthesis of the two repair markers through another molecular pathway.

### Example 2

### Fibroblast PGE2 Assay

The following example demonstrates that 8-prenylnarigenin can effectively reduce the basal levels of prostaglandin E₂ (PGE2) secreted by fibroblasts in vitro.

Primary human foreskin fibroblasts at passage 2 (P2) were seeded into 96-well plates at 10000 cells/well and maintained for 24 hours in an atmosphere of 5% carbon dioxide in Dulbeccos Modified Eagles Medium (DMEM) supplemented with 10% foetal calf serum. 8-prenylnaringenin was dissolved in ethanol was added to fresh cell media (final concentration 1% EtOH in DMEM, supplemented with 10% foetal calf serum) and added to the cells in the wells in triplicate and incubated for a further 24 hours. Phorbal myristate acetate (PMA) (Sigma) was added to the media and the cells incubated for a further 24 hours. PMA acts as the stressor that induces oxidative stress and inflammatory responses in cells. The fibroblasts/media were then analyzed as described below immediately or snap frozen in liquid nitrogen and stored at -70°C for future analysis.

### Prostaglandin E2 (PGE2) assay

Volumes of 50µl culture medium were taken for PGE2 assay after gently shaking the culture plate. PGE2 levels in the medium were determined with a Biotrak PGE2 immunoassay kit (Amersham, UK). The assay is based on the competition between unlabelled PGE2 in the sample and a fixed quantity of horseradish peroxidase labeled PGE2 for a limited amount of fixed PGE2 specific antibody. Concentrations of unlabelled sample PGE 2 are determined according to a standard curve, which was obtained at the same time.

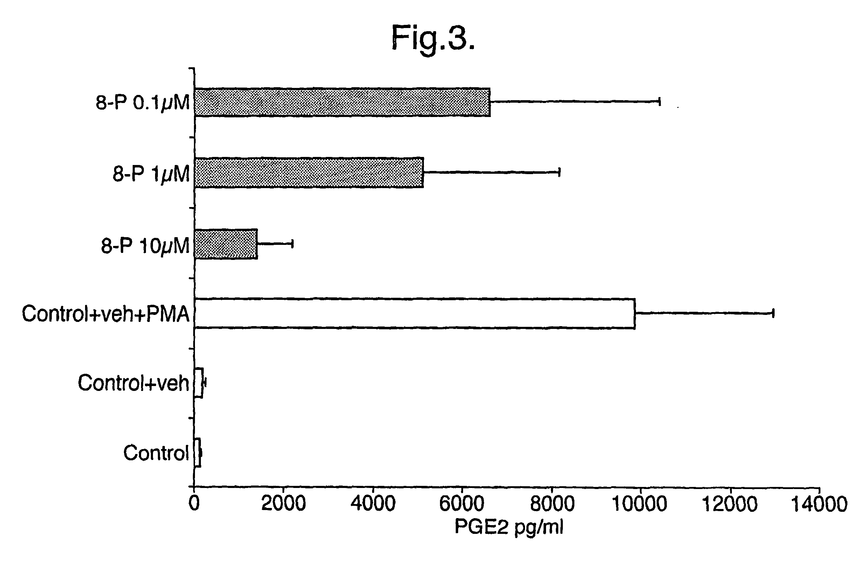

The anti-inflammatory potential of the test compounds was assessed by the ability of the compounds to reduce the basal levels of secreted PGE₂ as compared to the control. The results that were obtained are shown in Figure 3.

PGE₂ is a well known mediator of inflammation in the skin see Greaves *et al "Prostagludus, leukotriemes, phospholipase, platelet actuating factor and cytokines: an integrated approach* to *inflammation of human skin", Arch. Dermatol. Res. (1988) 280 [supp]: 533-541.* The results indicated that fibroblasts treated with 8-prenylnaringenin produce less of the pro-inflammatory prostaglandin PGE2, thereby reducing the inflammatory potential of the skin.

### Example 3

The formulation below describes an oil in water cream suitable for the methods and uses according to the present invention. The percentages indicated are by weight of the composition.

| | wt% |
|---|---|
| Mineral Oil | 4 |
| 8-prenylnaringenin | 1.15 |
| Brij 56* | 4 |
| Alfol 16RD* | 4 |
| Triethanolamine | 0.75 |
| Butane-1,3-diol | 3 |
| Xanthan gum | 0.3 |
| Perfume | Qs |
| Butylated hydroxy toluene | 0.01 |
| Water | To 100 |

| | |
|---|---|
| *Brij 56 is cetyl alcohol POE (10) Alfol 16RD is cetyl alcohol | |

### Example 4

The formulation below describes an emulsion cream according to the present invention.

| **FULL CHEMICAL NAME OR CTFA NAME** | **TRADE NAME** | **WT. %** |
|---|---|---|
| 8-prenylnaingenin | | 2.0 |
| disodium EDTA | Sequesterene Na2 | 0.05 |
| Magnesium aluminium silicate | Veegum Ultra | 0.6 |
| methyl paraben | Methyl Paraben | 0.15 |
| Simethicone | DC Antifoam Emulsion | 0.01 |
| butylene glycol 1,3 | Butylene Glycol 1,3 | 3.0 |
| Hydroxyethylcellulose | Natrosol 250HHR | 0.5 |
| Glycerine, USP | Glycerine USP | 2.0 |
| xanthan gum | Keltrol 1000 | 0.2 |
| Triethanolamine | Triethanolamine (99%) | 1.2 |
| stearic acid | Pristerene 4911 | 3.0 |
| propyl paraben NF | Propylparaben NF | 0.1 |
| glyceryl hydrostearate | Naturechem GMHS | 1.5 |
| stearyl alcohol | Lanette 18 DEO | 1.5 |
| Isostearyl palmitate | Protachem ISP | 6.0 |
| C12-15 alcohols octanoate | Hetester FAO | 3.0 |
| Dimethicone | Silicone Fluid 200 (50cts) | 1.0 |
| Cholesterol NF | Cholesterol NF | 0.5 |
| sorbitan stearate | Sorbitan Stearate | 1.0 |
| Butylated hydroxytoluene | Embanox BHT | 0.05 |
| Tocopheryl acetate | Vitamin E Acetate | 0.1 |
| PEG-100 stearate | Myrj 59 | 2.0 |
| sodium stearoyl lactylate | Pationic SSL | 0.5 |
| Hydroxycaprylic acid | Hydroxycaprylic Acid | 0.1 |
| retinyl palmitate | Vitamin A Palmitate | 0.06 |
| alpha-bisabolol | Alpha-bisabolol | 0.2 |
| water, DI | | q.s. to 100 |

Both the above topical compositions of example 3 and 4 may provide an effective cosmetic treatment to improve the appearance of wrinkled, aged, photo-damaged, and/or irritated skin, when applied to skin that has deteriorated through the aging or photoageing or when applied to youthful skin to help prevent or delay such deteriorative changes. The compositions can be processed in conventional manner.

## Claims

1. A topical composition comprising:
(a) 8-prenylnaringenin in an amount between 0.0001% and 50% by weight of the composition; and
(b) a dermatologically acceptable vehicle.

2. A topical composition according to claim 1 wherein the 8-prenylnaringenin is present at a level of 0.001 to 1.0% by weight of the composition.

3. A cosmetic method of providing at least one cosmetic skin care benefit selected from: treating/preventing wrinkling, sagging, dry, aged and/or photodamaged skin; boosting/maintaining collagen levels in skin; boosting/maintaining decorin levels in skin; improving skin texture, smoothness and/or firmness; and soothing irritated, red and/or sensitive skin; the method comprising applying to the skin a topical composition as claimed in claim 1 or 2.

4. Cosmetic use of a topical composition as claimed in any of claim 1 or claim 2 for providing at least one cosmetic skin care benefit selected from treating/preventing wrinkling, sagging, aged, dry, and/or photodamaged skin; boosting/maintaining collagen levels in skin; boosting/maintaining decorin levels in skin; soothing irritated, red and/or sensitive skin; improving skin texture, and smoothness and/or firmness.

## Patentansprüche

1. Topische Zusammensetzung umfassend:
(a) 8-Prenylnaringenin in einer Menge zwischen 0,0001 und 50 Gew.% der Zusammensetzung und
(b) ein dermatologisch akzeptables Vehikel.

2. Topische Zusammensetzung nach Anspruch 1, wobei das 8-Prenylnaringenin bei einem Niveau von 0,001 bis 1,0 Gew.-% der Zusammensetzung vorliegt.

3. Kosmetisches Verfahren zur Bereitstellung von zumindest einem kosmetischen Hautpflegevorteil, ausgewählt aus: Behandlung/Verhinderung von Falten, Durchhängen, trockener, gealterter und/oder lichtgeschädigter Haut; Verstärken/Halten von Kollagenniveaus in der Haut; Verstärken/Halten von Decorinniveaus in der Haut; Verbessern der Hauttextur, -weichheit und/oder -ebenheit; und Beruhigen gereizter, roter und/oder empfindlicher Haut, wobei das Verfahren die Auftragung einer topischen Zusammensetzung nach Anspruch 1 oder 2 auf die Haut umfaßt.

4. Kosmetische Verwendung einer topischen Zusammensetzung nach Anspruch 1 oder Anspruch 2 zur Bereitstellung von zumindest einem kosmetischen Hautpflegevorteil, ausgewählt aus Behandlung/Verhinderung von Falten, Durchhängen, trockener, gealterter und/oder lichtgeschädigter Haut; Verstärken/Halten von Kollagenniveaus in der Haut; Verstärken/Halten von Decorinniveaus in der Haut; Beruhigen gereizter, roter und/oder empfindlicher Haut; Verbessern der Hauttextur und -weichheit und/oder -ebenheit.

## Revendications

1. Composition topique comprenant :
(a) de la 8-prénylnaringénine en une proportion comprise entre 0,0001 % et 50 % en poids de la composition ; et
(b) un véhicule dermatologiquement acceptable.

2. Composition topique selon la revendication 1, dans laquelle la 8-prénylnaringénine est présente en une proportion de 0,001 à 1,0 % en poids de la composition.

3. Procédé cosmétique d'apport d'au moins un bienfait de soin cosmétique de la peau choisi parmi : le traitement/la prévention de la formation de rides, de la peau flasque, sèche, âgée et/ou photo-endommagée ; l'augmentation/le maintien des taux de collagène dans la peau ; l'augmentation/le maintien des taux de décorine dans la peau ; l'amélioration de la texture, du lissé et/ou de la fermeté de la peau ; et l'apaisement de la peau irritée, rougie et/ou sensible ; le procédé comprenant l'application sur la peau d'une composition topique telle que revendiquée dans la revendication 1 ou 2.

4. Utilisation cosmétique d'une composition topique telle que revendiquée dans la revendication 1 ou 2, pour l'apport d'au moins un bienfait de soin cosmétique de la peau choisi parmi le traitement/la prévention de la formation de rides, de la peau flasque, âgée, sèche et/ou photo-endommagée ; l'augmentation/le maintien des taux de collagène dans la peau ; l'augmentation/le maintien des taux de décorine dans la peau ; l'apaisement de la peau irritée, rougie et/ou sensible ; l'amélioration de la texture et du lissé et/ou de la fermeté de la peau.
